Europäisches Patentamt

European Patent Office

Office européen des brevets

(11) Numéro de publication : **0 348 292 B1**

## (12) FASCICULE DE BREVET EUROPEEN

(45) Date de publication du fascicule du brevet :
08.07.92 Bulletin 92/28

(51) Int. Cl.⁵ : **C07C 2/08**

(21) Numéro de dépôt : **89401727.6**

(22) Date de dépôt : **19.06.89**

(54) **Procédé catalytique de dimérisation, de codimérisation ou d'oligomérisation d'oléfines avec utilisation d'un fluide autogènede thermorégulation.**

(30) Priorité : **24.06.88 FR 8808633**

(43) Date de publication de la demande :
**27.12.89 Bulletin 89/52**

(45) Mention de la délivrance du brevet :
**08.07.92 Bulletin 92/28**

(84) Etats contractants désignés :
**CH DE GB IT LI NL**

(56) Documents cités :
**US-A- 2 222 304**
**US-A- 4 709 111**

(73) Titulaire : **INSTITUT FRANCAIS DU PETROLE**
**4, Avenue de Bois Préau**
**F-92502 Rueil-Malmaison (FR)**

(72) Inventeur : **Dang Vu, Quang**
**48 bis, Boulevard du Général Leclerc**
**F-92200 Neuilly (FR)**
Inventeur : **Chauvin, Yves**
**64, avenue du Général Leclerc**
**F-78230 Le Pecq (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 348 292 B1

Jouve, 18, rue Saint-Denis, 75001 PARIS

## Description

L'invention concerne un procédé pour effectuer, généralement sous pression, des réactions de dimérisation, de codimérisation et d'oligomérisation (sélectives notamment) d'oléfines, en présence d'au moins un catalyseur, habituellement solide, dans au moins une zone réactionnelle dont la témperature est contrôlée par un dispositif d'échange thermique à base de plaques creuses disposé en son sein.

Généralement dans ce type de réactions, lorsqu'on opère selon l'invention, au moins un des réactifs se trouve soit à l'état liquide, soit dans un état rendant possible sa circulation par une pompe (état supercritique), tel que le rapport Tr entre le température T (en kelvin) du système des réactifs et la température (pseudo-) critique Tc (en kelvin) dudit système soit de préférence inférieur à 2, par exemple à 1,5.

L'oléfine employée peut notamment être choisie parmi l'éthylène, le propylène, le styrène, l'un des isomères des butènes ou un de leurs mélanges. Cette oléfine peut être mise en oeuvre à l'état pur ou en mélange avec un ou plusieurs composés ne réagissant pas sur le catalyseur dans les conditions utilisées, tels que par exemple les hydrocarbures saturés cycliques ou acyliques, en particulier ceux ayant de 2 a 10 atomes de carbone. La concentration de l'oléfine dans le mélange peut être de 5 à 100 % de préférence de 10 à 100 % en poids.

L'invention concerne plus particulièrement un procédé de dimérisation sélective du propylène en méthyl-4 pentène-1, par exemple au moyen de catalyseurs solides à base de potassium et/ou de sodium.

Il est connu de dimériser ou d'oligomériser des oléfines en phase liquide homogène pour obtenir, par exemple, des dimates en C6 (brevets de la demanderesse US-A-4283305 et 4366087).

Il est beaucoup plus difficile de faire la dimérisation sélective quand on met en oeuvre un catalyseur hétérogène, par exemple pour dimériser le propylène en méthyl-4 pentène-1, avec une sélectivité suffisante, notamment supérieure à 85 %. Il faut, en effet, alors maintenir dans des limites assez étroites, une température relativement constante en-dessous de laquelle l'activité du catalyseur tombe à une valeur qui rend la réaction inexploitable industriellement et au-dessus de laquelle interviennent des réactions consécutives d'isomérisation qui abaissent la sélectivité et entraînent des problèmes de séparation quasi-insurmontables.

Le système réactionnel isotherme le plus souvent utilisé est le réacteur à tubes-calandre monopasse où l'intérieur des tubes est rempli de catalyseur et constitue ainsi le milieu réactionnel. Mais, en dimérisation sélective, le système catalytique subit en général une désactivation spontanée et/ou accidentelle (due aux impuretés), ce qui nécessite le renouvellement périodique de ce catalyseur, contrainte techniquement difficile à mettre en oeuvre avec un réacteur dans lequel les tubes en nombre important doivent être remplis et vidés un à un de façon manuelle.

Il est également connu, lorsque la température de la réaction doit être maintenue dans des limites relativement étroites, de placer au sein du lit catalytique un appareil de transfert thermique soit à base de tubes (GB-B2046618), soit à base de plaques (US-A-3666423) soit à base de grilles (US-A- 4693807), et de faire circuler à l'intérieur de cet appareil un fluide destiné au transfert thermique et communément désigné sous le nom de fluide de thermorégulation.

L'inconvénient dans l'utilisation d'un appareil de transfert thermique à base de tubes provient du fait que la liaison entre ces tubes individuels est très encombrante et que, par conséquent, le montage de l'ensemble est très difficile à réaliser correctement a l'intérieur du réacteur. L'inconvénient de l'appareil de transfert thermique à base de plaques du brevet US-A-3666423 est son encombrement et sa faible efficacité. Pour pouvoir résister à la pression réactionnelle, les plaques ne sont que partiellement évidées et le fluide de thermorégulation ne dispose ainsi que d'une faible partie de la surface des plaques pour faire son travail d'échange.

Dans son brevet US-A-4544544, la demanderesse a proposé, pour des systèmes à réactifs gazeux, un procédé permettant d'utiliser des plaques creuses, en tôles minces et à section interne rectangulaire.

Les plaques utilisées dans les procédés selon la présente invention travaillent très peu à la contrainte, ce qui permet de les évider totalement et de laisser le fluide de thermorégulation assurer l'échange à travers la totalité de la surface disponible. De plus, le montage et les connexions sont suffisamment simples pour être réalisés facilement dans l'espace restreint offert par le réacteur.

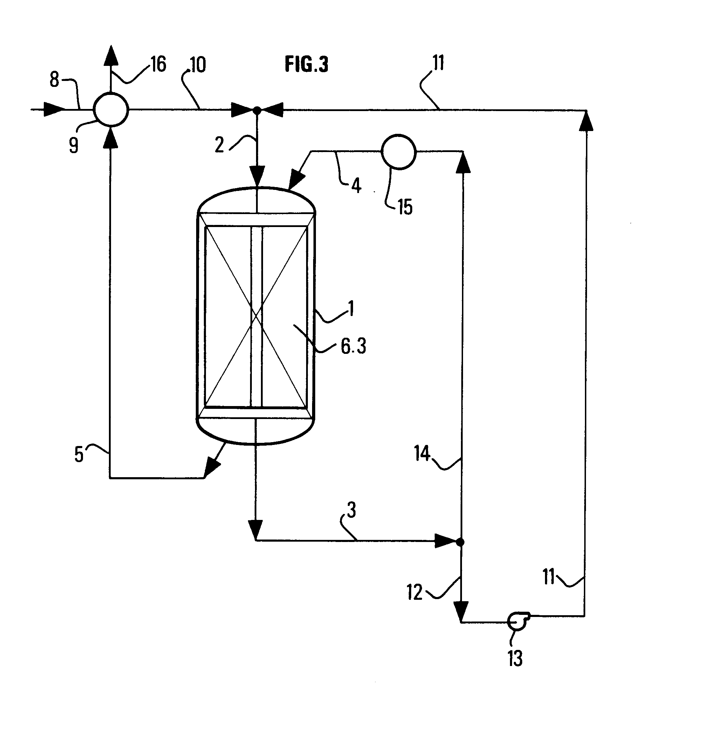

La présente invention utilise un système réactionnel monoétagé continu, équipé d'un appareil à plaques de transfert thermique. La figure 3 explique la marche de l'unité :

– le fluide réactionnel va d'abord être employé comme fluide de thermorégulation de l'unité et du lit de catalyseur ; ensuite, il traversera le lit de catalyseur solide enfermé dans une enveloppe (1) de forme sensiblement cylindrique et allongée ;

– le sein du lit catalytique est refroidi par le cheminement du fluide de thermorégulation qui circule dans un dispositif d'échange thermique plongé dans ledit lit catalytique et à base de plaques creuses fabriquées, par exemple, à partir de tôles minces plates ou ondulées comme il sera expliqué plus loin ;

– le fluide de thermorégulation circulant à l'intérieur des plaques comprend le(s) réactif(s) constituant la charge fraîche, la circulation de ce fluide étant habituellement assurée par au moins une pompe (13) ;

– le système de thermorégulation est un système ouvert sur le système réactionnel ; en effet, il est alimenté en continu par le(s) réactif(s) d'appoint qui passe(nt) directement du système de thermorégulation dans le réacteur sans barrière mécanique permanente ou transitoire.

Selon le procédé de l'invention, on envoie la charge fraîche (par exemple du propylène liquide), sous une pression généralement comprise entre 1,2 et 12 MPa, dans le conduit 8 de l'unité de fabrication (par exemple du méthyl-4 pentène-1).

Cette charge fraîche est d'abord de préférence indirectement préchauffée par de l'effluent réactionnel chaud (provenant de la conduite 5) à travers l'échangeur de chaleur 9. ELle passe ensuite dans le conduit 10 et va à la rencontre d'une fraction recyclée du fluide de thermorégulation que l'on appellera "charge recyclée" par la suite, cette fraction recyclée étant amenée par le conduit 11.

Le rapport en poids entre la charge recyclée et la charge fraîche est utilement compris entre 1 et 500, de préférence entre 2 et 200, et de manière encore plus préférée entre 5 et 100.

Le mélange ainsi obtenu (charge fraîche + charge recyclée) forme le fluide de thermorégulation (ou fluide caloporteur) du réacteur (1). Ce fluide, qui se trouve en général entre 100 et 200°C, de préférence aux alentours de 150°C, et à une température réduite d'environ 1,13, et dans un état tel qu'il peut toujours être véhiculé par une pompe, pénètre par le conduit 2 dans l'intérieur des plaques réfrigérantes creuses (6.3) disposées au sein du lit catalytique contenu dans le réacteur (1).

Le fluide de thermorégulation absorbe la chaleur de réaction dégagée au fur et à mesure de la formation du produit de synthèse (par exemple le methyl-4 pentène-1 obtenu par dimérisation sélective du propylène). Ce fluide caloporteur sort du réacteur (1) par la conduite 3.

Il est alors séparé en deux portions. Un première portion forme la charge à traiter qui va circuler à travers le lit de catalyseur. Cette charge sera par la suite appelée "charge d'appoint". Elle est envoyée vers le réacteur (1) par la conduite 14. Une deuxième portion du fluide de thermorégulation soutirée par la conduite 3 sera recyclée par la conduite 11 dans les plaques échangeuses de chaleur 6.3 (en mélange avec de la charge fraîche en provenance de la conduite 10). Cette deuxième portion de fluide de thermorégulation est nommée "charge recyclée".

La "charge recyclée" passe dans le conduit 12 où elle est aspirée par la pompe 13 (de préférence centrifuge) pour être injectée dans la conduite 11 déjà mentionnée.

Selon les caractéristiques de l'invention, le rapport en poids entre la charge recyclée et la charge d'appoint est avantageusement compris entre 1 et 500, de préférence entre 2 et 200 et de manière encore plus préférée entre 5 et 100. Un rapport trop faible interdit un contrôle thermique satisfaisant au sein du lit catalytique ; un rapport trop élevé demande une section de passage telle que les plaques creuses deviennent trop épaisses et trop encombrantes.

La "charge d'appoint", non reprise par la pompe 13, passe dans le conduit 14 à travers l'échangeur 15 dont le rôle est de réajuster au niveau voulu la température à l'entrée du réacteur 1. Cette charge d'appoint sort de l'échangeur 15 par la conduite 4 et traverse ensuite le lit catalytique contenu dans le réacteur 1. L'effluent réactionnel résultant est évacué dudit réacteur par la conduite 5 pour venir préchauffer la charge fraîche à travers l'échangeur 9. De 9, ledit effluent est utilement dirigé, par le conduit 16, sur une unité de distillation et de conditionnement (non représentée sur la figure).

Lorsqu'il s'agit de dimériser le propylène de manière sélective en méthyl-4 pentène 1, on utilise par exemple un catalyseur à base de carbonate alcalin imprégné de préférence par le métal de la même famille.

Grâce au bon contrôle thermique assuré par le système de réfrigération à plaques, la conversion a pu être poussée très loin sans que la sélectivité de l'opération en soit affectée.

Ainsi, l'invention a pour objet un procédé catalytique de dimérisation ou de codimérisation ou d'oligomérisaiton d'oléfines effectué habituellement sous pression, en présence d'un catalyseur solide, dans une zone de réaction définie par une enceinte de forme sensiblement cylindrique et dont la section est sensiblement circulaire, ladite enceinte renfermant un lit généralement fixe de catalyseur dans lequel est plongée une pluralité d'espaces internes creux, dans lesquels circule un fluide de thermorégulation, sous une pression sensiblement égale à la pression à laquelle est soumis le mélange réactionnel, procédé dans lequel:

– on introduit une charge fraîche liquide, contenant au moins une oléfine, en mélange avec une charge recyclée définie ci-dessous, dans lesdits espaces internes creux délimités par des parois, le rapport en poids entre la charge recyclée et la charge fraîche étant compris entre 1 et 500, le mélange charge fraîche-charge recyclée formant le fluide de thermorégulation ;

– on soutire le fluide de thermorégulation desdits espaces internes et on sépare ce fluide en deux portions appelées ci-après charge recyclée et charge d'appoint, le rapport en poids entre ladite charge recyclée et ladite charge d'appoint étant compris entre 1 et 500 ;

– on renvoie ladite charge recyclée vers lesdits espaces internes à titre de composant du fluide de thermorégulation ;
– on envoie ladite charge d'appoint dans le lit de catalyseur ;
– on soutire un effluent réactionnel du lit de catalyseur.

L'invention concerne également la mise en oeuvre dudit procédé avec l'utilisation d'espaces internes creux de conception particulière pour la circulation du fluide de thermorégulation.

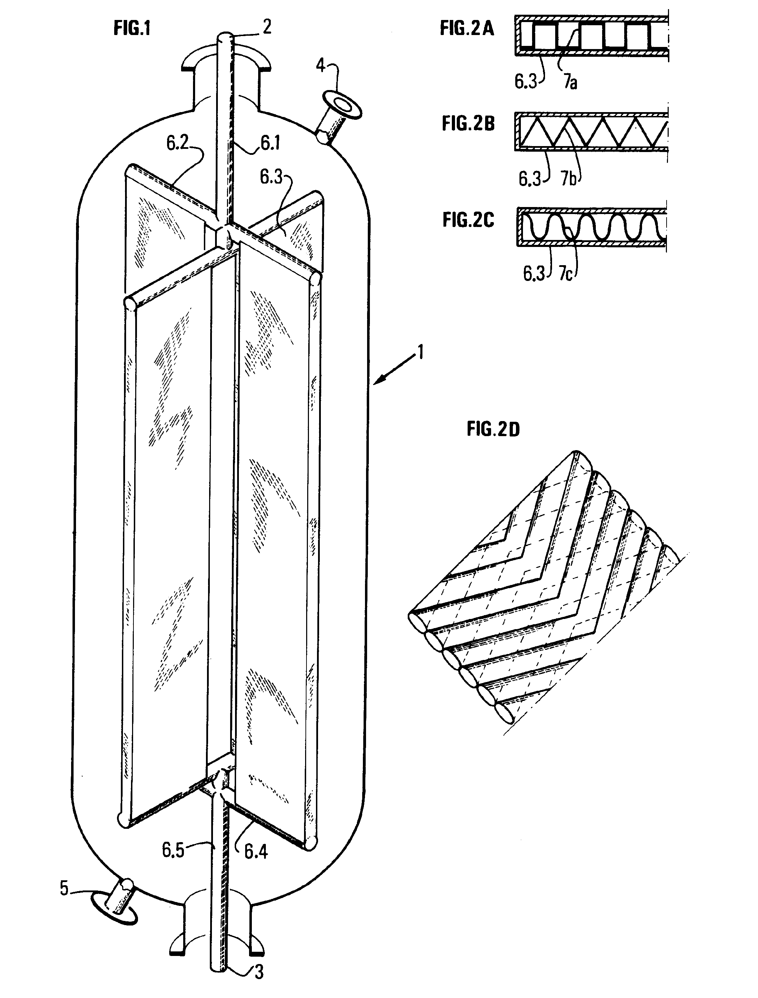

Dans la figure 1 les espaces internes creux (ou plaques) ont des faces planes. Les figures 2A, 2B, 2C et 2D représentent des plaques selon un perfectionnement de l'invention.

Dans la figure 1 est dessinée une enceinte (1), de forme sensiblement cylindrique et dont la section en coupe a une forme sensiblement circulaire, comportant au moins une conduite (2) pour l'introduction d'un fluide de thermorégulation, au moins une conduite (3) pour le soutirage dudit fluide, au moins une conduite (4) pour l'introduction d'une charge dite d'appoint dans l'enceinte et au moins une conduite (5) pour le soutirage de l'effluent réactionnel de ladite enceinte. L'enceinte (1) renferme en outre :

a) au moins un collecteur distributeur central (6.1), par exemple vertical, dont l'axe correspond généralement à l'axe de l'enceinte, qui est situé dans la partie supérieure de l'enceinte et est connecté à la conduite 2,

b) une pluralité de collecteurs distributeurs (6.2), perpendiculaires à l'axe de l'enceinte, ces collecteurs étant connectés individuellement au collecteur distributeur central (6.1),

c) au moins un collecteur receveur central (6.5), par exemple vertical, dont l'axe correspond en général à l'axe de l'enceinte, qui est situé dans la partie inférieure de l'enceinte et est connecté à la conduite 3,

d) une pluralité de collecteurs receveurs (6.4), perpendiculaires à l'exe de l'enceinte, ces collecteurs étant connectés individuellement au collecteur receveur central (6.5),

e) une pluralité de plaques creuses, continues et allongées destinées à la circulation du fluide de thermorégulation, chaque plaque comportant une ouverture sur un collecteur distributeur (6.2) et une ouverture sur un collecteur receveur (6.4).

Les faces desdites plaques creuses peuvent être constituées par des tôles ondulées dont les ondulations ont au choix l'une des formes suivantes : carrée, rectangulaire, triangulaire, sinusoïdale et en chevrons (voir figure 2 D), le but étant de créer une forte turbulence sur l'écoulement du fluide de thermorégulation.

Il convient que lesdites plaques creuses soient sensiblement parallélépipèdiques (6.3), chaque plaque comportant deux faces larges parallèles délimitant un plan disposé radialement par rapport à l'axe de l'enceinte et quatre faces minces, deux d'entre elles étant parallèles à l'axe, chaque plaque étant connectée, par sa face mince supérieure perpendiculaire à l'axe de l'enceinte, à un collecteur distributeur (6.2), et, par sa face mince inférieure perpendiculaire à l'axe de l'enceinte, à un collecteur receveur (6.4). Ces quatre faces minces peuvent éventuellement être non planes, mais par exemple semi-cylindriques.

A noter qu'on peut alors aménager dans chacune desdites plaques creuses sensiblement parallélépipèdiques des canaux adjacents au moyen de tôles ondulées afin d'améliorer la circulation du fluide de thermorégulation , les sections desdits canaux ayant au choix l'une des formes suivantes : carrée, rectangulaire (voir figure 2A), triangulaire (voir figure 2B), sinusoidale (voir figure 2C), ces canaux reliant entre elles les deux faces minces perpendiculaires à l'axe de l'enceinte d'une même plaque.

Selon une variante de l'invention, les plaques creuses peuvent éventuellement avoir des largeurs différentes, ce qui permet de maintenir un rapport minimum entre le volume de l'enceinte et la surface d'échange, tout en évitant une trop grande distance entre un point quelconque de l'enceinte et la plaque la plus proche.

Les tôles éventuellement utilisées dans les divers modes de réalisation de l'invention ont, généralement, moins de 10 millimètres d'épaisseur, de préférence moins de 3 millimètres d'épaisseur.

Dans la figure 1, donnée à titre d'exemple, on décrira le trajet du fluide de thermorégulation à travers des plaques creuses (ou espaces internes creux) sensiblement parallélépidèdiques. La charge, sous forme de charge d'appoint, pénètre dans l'enceinte (1) par la conduite 4, passe à travers le lit catalytique contenu dans ladite enceinte, puis elle sort de ladite enceinte, sous forme d'effluent réactionnel, par la conduite 5. Le fluide de thermorégulation autogène (c'est-à-dire constitué par un ou plusieurs des composantes constituant la charge fraîche), passe du conduit 2 dans le collecteur distributeur central (6.1). Il se répartit ensuite dans les collecteurs distributeurs (6.2). Puis il pénètre dans chacune des plaques creuses (6.3) par leur face mince supérieure perpendiculaire à l'axe de l'enceinte, ces plaques creuses étant disposées au sein du lit catalytique contenu dans l'enceinte (1). Le fluide descend à l'intérieur desdites plaques creuses sous la forme d'une nappe. A la sortie des plaques creuses, il est collecté dans les collecteurs receveurs (6.4) qui sont connectés individuellement au collecteur receveur central (6.5) dans lequel le fluide passe ensuite. Enfin, le fluide de thermorégulation sort par la conduite (3).

L'avantage d'un fluide de thermorégulation autogène est, d'une part, qu'il n'y a pas de différence de pression entre l'intérieur et l'extérieur des plaques (à part celle crée par les pertes de charge dues à la circulation des différents fluides) et, d'autre part, qu'en cas de fuite, il n'y a pas de danger de pollution du système catalytique.

En bref, dans l'invention perfectionée :

– on envoie le fluide de thermorégulation, autogène (formé par le(s) composant(s) constituant la charge fraîche (et donc la charge recyclée)), à travers une zone de distribution centrale (6.1),

– on répartit ledit fluide en provenance de la zone de distribution centrale (6.1) dans des zones distributives (6.2),

– on envoie ledit fluide, depuis lesdites zones distributives (6.2), dans lesdits espaces internes creux (6.3), délimités par des parois (lesdits espaces ayant une forme sensiblement parallélépipèdique), chaque espace (6.3) comportant alors deux faces larges parallèles délimitant un plan disposé radialement par rapport à l'axe de la zone de réaction (1) et quatre faces minces, deux d'entre elles étant parallèles à l'axe de la zone de réaction (1), les deux autres étant perpendiculaires à cet axe), par leur face mince supérieure perpendiculaire à l'axe de la zone de réaction (1),

– on fait circuler ledit fluide à l'intérieur desdits espaces internes creux (6.3) sous la forme d'une nappe,

– on évacue ledit fluide de thermorégulation desdits espaces internes creux (6.3), par leur face mince inférieure perpendiculaire à l'axe de la zone de réaction (1), dans des zones collectrices (6.4) qui sont reliés à une zone de collecte centrale (6.5) par laquelle on soutire ensuite ledit fluide.

Sur les figures 1 et 3 l'enceinte (1) est représentée en position sensiblement verticale : les circulations du fluide de thermorégulation et de la charge d'appoint peuvent se faire de haut en bas comme décrit précédemment, mais également de bas en haut (et donc aussi à contre-courant).

De plus, sur les figures 1 et 3, on a arbitrairement représenté la conduite 4 d'admission de la charge (d'appoint) au sommet de l'enceinte 1 et la conduite 5 de soutirage de l'effluent réactionnel à la base de l'enceinte 1, mais ces conduites (4 et 5) peuvent en fait se situer à tout niveau adéquat de l'enceinte.

La figure 1 représente un (e) réacteur (enceinte) axial(e) dans lequel (laquelle) les réactifs traversent le lit de catalyseur de façon parallèle à l'axe du réacteur.

L'invention peut s'appliquer également à un réacteur radial comportant un panier perméable de la forme d'un anneau cylindrique, par exemple délimité par deux cylindres coaxiaux, dans lequel sont disposés le catalyseur et les plaques creuses et où les réactifs traversent le lit perpendiculairement à l'axe du réacteur.

Les figures 2A, 2B et 2C représentent trois plaques creuses sensiblement parallélépipédiques (6.3) dans lesquelles on a aménagé des canaux adjacents (7a, 7b, et 7c) au moyen de tôles ondulées, les sections desdits canaux ayant au choix l'une des formes suivantes : carrée, rectangulaire (7a), triangulaire (7b) et sinusoïdale (7c), ces canaux reliant entre elles les deux faces minces perpendiculaires à l'axe de l'enceinte d'une même plaque : d'une part, la présence de ces canaux adjacents assure la solidité des plaques creuses (6.3) qui peuvent atteindre et dépasser, par dix mètre de hauteur, d'autre part, elle évite la formation de zones mortes qui pourraient se former du fait de l'écoulement en nappe du fluide de thermorégulation à l'intérieur des plaques.

L'assemblage des tôles peut être réalisé soit par soudure, soit beaucoup plus économiquement par brasure soit par points, soit par immersion dans un bain, ou toute autre technique adéquate.

L'invention est illustrée par les exemples suivants :

Exemple 1 (selon l'invention)

Dans un réacteur cylindrique vertical, de 0,5 mètre de diamètre, équipé d'un système de thermorégulation à plaques creuses conforme aux figures 1 et 2A, on dispose du catalyseur obtenu par dépôt de 3,5 % en poids de sodium sur des écailles de carbonate de potassium lié par 1,5 % de graphite et préalablement activé à 230°C pendant 3 heures.

Puis, on remplit sous une pression de 9 MPa, l'unité représentée selon la figure 3 (par le conduit 8) avec du propane et on assure au moyen de la pompe 13 une recirculation de 300 m³/h.

Au moyen du réchauffeur extérieur à vapeur 15 on élève progressivement la température du propane. Quand cette température atteint 150°C environ, on introduit du propylène dans l'unité (par le conduit 8), tout en purgeant le propane par le conduit 16.

Au bout de quelques heures, il s'établit un régime stationnaire avec un débit de propylène de 5 m³/h une conversion du propylène de 28,3 % et une sélectivité en méthyl-4 pentène 1 de 89,1 %. Ce régime a pu être maintenu pendant plusieurs centaines d'heures sans variation sensible de la conversion du propylène et de la sélectivité en méthul-4 pentène-1.

Exemple 2 (comparatif)

On utilise le même catalyseur que dans l'exemple 1, que l'on dispose dans le même réacteur d'où on a enlevé le système de thermorégulation selon l'invention, le débit horaire de propylène étant identique.

La température d'alimentation du réacteur en propylène est de 130°C. La température de l'effluent réactionnel à la sortie du réacteur est de 190°C.

On note que la conversion du propylène ne dépasse pas 15,0 % et la sélectivité en méthyl-4 pentène 1 n'atteint que 65,1 %, la majeure partie des sous-produits de la réaction étant formée par du méthyl-4 pentène-2. On constate de plus qu'après une cinquantaine d'heures le catalyseur a perdu une grande partie de son activité.

## Revendications

1. Procédé catalytique de dimérisation ou de codimérisation ou d'oligomérisation d'oléfines effectué sous pression, en présence d'un catalyseur solide, dans une zone de réaction définie par une enceinte de forme sensiblement cylindrique et dont la section est sensiblement circulaire, ladite enceinte renfermant un lit de catalyseur dans lequel est plongée une pluralité d'espaces internes creux sensiblement de forme parallélépipèdique dans lequels circule un fluide de thermorégulation, sous une pression sensiblement égale à la pression à laquelle est soumis le mélange réactionnel, procédé dans lequel :
– on introduit une charge fraîche liquide, contenant au moins une oléfine, en mélange avec une charge recyclée définie ci-dessous, dans lesdits espaces internes creux délimités par des parois, le rapport en poids entre la charge recyclée et la charge fraîche étant compris entre 1 et 500, le mélange charge fraîche-charge recyclée formant le fluide de thermorégulation ;
– on soutire le fluide de thermorégulation desdits espaces internes et on sépare ce fluide en deux portions appelés ci-après charge recyclée et charge d'appoint, le rapport en poids entre ladite charge recyclée et ladite charge d'appoint étant compris entre 1 et 500 ;
– on renvoie ladite charge recyclée vers lesdits espaces internes à titre de composant du fluide de thermorégulation ;
– on envoie ladite charge d'appoint dans ledit lit de catalyseur ;
– on soutire un effluent réactionnel dudit lit de catalyseur.

2. Procédé selon la revendication 1 dans lequel ladite charge fraîche est préchauffée par contact indirect avec l'effluent réactionnel.

3. Procédé selon l'une des revendications 1 et 2 dans lequel le rapport en poids entre ladite charge recyclée et ladite charge fraîche est compris entre 2 et 200.

4. Procédé selon l'une des revendications 1 à 3 dans lequel le rapport en poids entre ladite charge recyclée et ladite charge d'appoint est compris entre 2 et 200.

5. Procédé selon l'une des revendications 1 à 4 dans lequel :
– on envoie le fluide de thermorégulation dans une zone de distribution centrale,
– on répartit le fluide en provenance de la zone de distribution centrale dans des zones distributives,
– on envoie ledit fluide, depuis lesdites zones distributives, dans lesdits espaces internes creux délimités par des parois, chaque espace interne creux ayant sensiblement une forme parallélépipèdique, par leur face mince supérieure perpendiculaire à l'axe de ladite zone de réaction,
– on fait circuler ledit fluide à l'intérieur desdits espaces internes creux sous la forme d'une nappe,
– on évacue ledit fluide de thermorégulation desdits espaces internes creux, par leur face mince inférieure perpendiculaire à l'axe de la zone de réaction, dans des zones collectrices qui sont reliées à une zone de collecte centrale par laquelle on soutire ensuite ledit fluide.

6. Procédé selon l'une des revendications 1 à 5 appliqué à la dimérisation sélective du propylène en méthyl-4 pentène-1.

## Patentansprüche

1. Katalytisches Verfahren zum Dimerisieren oder Codimerisieren oder Oligomerisieren von Olefinen, ausgeführt unter Druck, in Anwesenheit eines festen Katalysators, in einer Reaktionszone, die durch einen umschlossenen Raum im wesentlichen zylindrischer Gestalt definiert und deren Querschnitt im wesentlichen kreisförmig ist, wobei dieser umschlossene Raum ein Katalysatorbett umschließt, in welches eine Vielzahl von hohlen Innenräumen im wesentlichen parallel epipedförmiger Gestalt taucht, in welchen ein Wärmeregulierungsfluid unter einem Druck zirkuliert, der im wesentlichen gleich dem Druck ist, dem das Reaktionsgemisch ausgesetzt wird, Verfahren, bei dem:
– man eine frische flüssige wenigstens ein Olefin enthaltende Charge im Gemisch mit einer unten definierten recyclisierten Charge in diese durch Wandungen begrenzten hohlen Innenräume einführt, wobei das Gewichtsverhältnis zwischen der recyclisierten Charge und der frischen Charge zwischen 1 und 500 beträgt und das Gemisch frische Charge-recyclisierte Charge das Wärmeregulierungsfluid bildet;
– man das Wärmeregulierungsfluid aus diesen Innenräumen abzieht und dieses Fluid in zwei Teile, im folgenden recyclisierte Charge und Zuführcharge genannt, teilt, wobei das Gewichtsverhältnis zwischen dieser recyclisierten Charge und dieser Zuführcharge zwischen 1 und 500 beträgt;
– man diese recyclisierte Charge gegen diese Innenräume als Wärmeregulierungsfluidkomponente schickt;
– man diese Zuführcharge in dieses Katalysatorbett gibt; und
– man einen Reaktionsabstrom aus diesem Katalysatorbett abzieht.

2. Verfahren nach Anspruch 1, bei dem diese frische Charge im indirekten Kontakt mit dem Reaktionsabstrom vorgewärmt wird.

3. Verfahren nach einem der Ansprüche 1 und 2, bei dem das Gewichtsverhältnis zwischen dieser recyclisierten Charge und dieser frischen Charge zwischen 2 und 200 beträgt.

4. Verfahren nach einem der Ansprüche 1 bis 3, bei dem das Gewichtsverhältnis zwischen dieser recyclisierten Charge und dieser Zuführcharge zwischen 2 und 200 beträgt.

5. Verfahren nach einem der Ansprüche 1 bis 4, bei dem:
– man das Wärmeregulierungsfluid in eine zentrale Verteilerzone schickt,
– man das aus dieser zentralen Verteilerzone stammende Fluid in verteilende Zonen aufteilt,
– man dieses Fluid aus diesen aufteilenden Zonen in diese durch Wandungen begrenzten hohlen Innenräume - wobei jeder hohle Innenraum im wesentlichen eine parallel epipedförmige Form hat - über ihre obere Schmalseite senkrecht zur Achse dieser Reaktionszone, schickt
– man dieses Fluid im Inneren dieser hohlen Innenräume in Form einer Bahn zirkulieren läßt, und
– man dieses Wärmeregulierungsfluid aus diesen hohlen Innenräumen über ihre untere Schmalseite senkrecht zur Achse der Reaktionszone in Sammlerzonen evakuiert, die mit einer zentralen Sammlerzone verbunden sind, über die man dann dieses Fluid abzieht.

6. Verfahren nach einem der Ansprüche 1 bis 5, angewendet auf die selektive Dimerisierung des Propylens in 4-Methyl-1-penten.

**Claims**

1. A catalytic method for dimerizing or codimerizing or oligomerizing olefins, carried out under pressure, in the presence of a solid catalyst, in a reaction zone defined by a substantially cylindrical enclosure whose section is substantially circular, said enclosure containing a catalyst bed in which a plurality of hollow internal spaces having a substantially parallelepipedic shape is disposed, through which flows a thermoregulation fluid, at a pressure substantially equal to the pressure to which the reaction mixture is subjected, in which method :
– a fresh liquid charge , containing at least one olefin, mixed with a recycled charge defined below, is introduced into said hollow internal spaces defined by walls, the weight ratio between the recycled charge and the fresh charge being between 1 and 500, the fresh charge-recycled charge mixture forming the thermoregulation fluid;

– the thermoregulation fluid is drawn off from said internal spaces and this fluid is separated into two portions called hereafter recycled charge and make-up charge, the weight ratio between said recycled charge and said make-up charge being between 1 and 500;
– said recycled charge is fed into said internal spaces as thermoregulation fluid component;
– said make-up charge is fed into said catalyst bed;
– a reaction effluent is drawn off from said catalyst bed.

2. A method according to claim 1 wherein said fresh charge is preheated by indirect contact with the reaction effluent.

3. A method according to one of claims 1 and 2 wherein the weight ratio between said recycled charge and said fresh charge is between 2 and 200.

4. A method according to one of claims 1 to 3 wherein the weight ratio between said recycled charge and said make-up charge is between 2 and 200.

5. A method according to one of claims 1 to 4 wherein :
– the thermoregulation fluid is fed into a central distributing zone,
– the fluid from the central distributing zone is divided among distributing zones ,
– said fluid is fed,from said distributing zones,into said hollow internal spaces defined by walls, each hollow internal space having substantially a parallelepipedic shape, through their thin upper face perpendicular to the axis of said reaction zone,
– said fluid is caused to flow inside said hollow internal spaces in the form of a sheet,
– said thermoregulation fluid is discharged from said hollow internal spaces, through their lower thin face perpendicular to the axis of the reaction zone, into collecting zones which are connected to a central collecting zone through which said fluid is then drawn off.

6. A method according to one of claims 1 to 5 applied to the selective dimerization of propylene into methyl-4 pentene-1.

**FIG.1**

2

4

6.2   6.1

6.3

1

**FIG.2A**

6.3   7a

**FIG.2B**

6.3   7b

**FIG.2C**

6.3   7c

**FIG.2D**

6.5   6.4

5

3

FIG.3